# EUROPEAN PATENT SPECIFICATION

(11) **EP 4 172 009 B1**
(45) Date of publication and mention of the grant of the patent: **01.01.2025**
(21) Application number: 21748657.0
(22) Date of filing: 30.06.2021
(51) Int. Cl.: B60S 1/64, A61L 9/14, B60H 3/00

(54) **CAB PROVIDED WITH A SANITIZATION SYSTEM**
KABINE MIT EINEM DESINFEKTIONSSYSTEM
CABINE POURVUE D'UN SYSTÈME DE DÉSINFECTION

(30) Priority: 30.06.2020 IT 202000015769
(43) Date of publication of application: 03.05.2023
(73) Proprietor: IVECO S.P.A., 10156 Torino (IT)
(72) Inventor: AIMO BOOT, Marco, 10072 CASELLE TORINESE (TO) (IT)
(74) Representative: Studio Torta S.p.A.
(86) International application number: PCT/IB2021/055875
(87) International publication number: WO 2022/003599

(56) References cited:
- EP-A1- 2 353 902
- WO-A1-2015/177437
- FR-A1- 2 999 482
- US-A1- 2008 153 408

## Description

### TECHNICAL FIELD

This invention relates to a vehicle according to the preamble of claim 1 and to an associated method.

### STATE OF THE PRIOR ART

It is usual for companies transporting goods or heavy equipment to have fleets of vehicles that are used interchangeably by different drivers depending on the job assigned to them.

The current health emergency, due to the SARS-COV-2 pandemic, has made the need to sanitize the cabs of heavy vehicles, such as trucks that may be used by different drivers as in the above example, topical.

However, current sanitizing methods involve manual sanitizing of the cabs of such vehicles, which wastes time and sanitizing products and consequently adds to costs. In addition, users engaged in this sanitization practice, who are often the same drivers who are required to disinfect the seat at the end of their shift, are exposed to the use of potentially hazardous chemicals.

Clearly, when the sanitizing operation is currently carried out manually, there is a risk of not performing optimal sanitization due to the intrinsic quality of the sanitization linked to the action of the operator, who may forget to sanitize some parts or sanitize them in a careless fashion, putting the health of the driver, who will be called to take over, at risk.

Examples of known sanitizing systems in the art are disclosed in publications EP2353902 A1, WO2015177437 A1, FR2999482 A1 or US2008153408 A1.

There is a need, therefore, to optimally sanitise the cabs of heavy-duty vehicles without wasting time or increasing costs.

The purpose of this invention is to fulfil the above requirements in an optimal and economical manner.

### SUMMARY OF THE INVENTION

The above-mentioned purpose is achieved with a vehicle comprising a sanitization system and a related method as claimed in the attached independent claims.

Additional, preferred embodiments of the invention are produced according to the dependent claims or the claims related to the above-mentioned independent claims.

### BRIEF DESCRIPTION OF THE DRAWINGS

In order to better understand this invention, a preferred embodiment thereof will now be described by way of non-limiting example and with reference to the accompanying drawings, in which:
- Figure 1 is a schematic perspective view illustrating a sanitization system of a vehicle according to the invention; and
- Figure 2 is a schematic side view in partial cross-section of a portion of a vehicle comprising a sanitization system according to the invention.

### DETAILED DESCRIPTION OF THE INVENTION

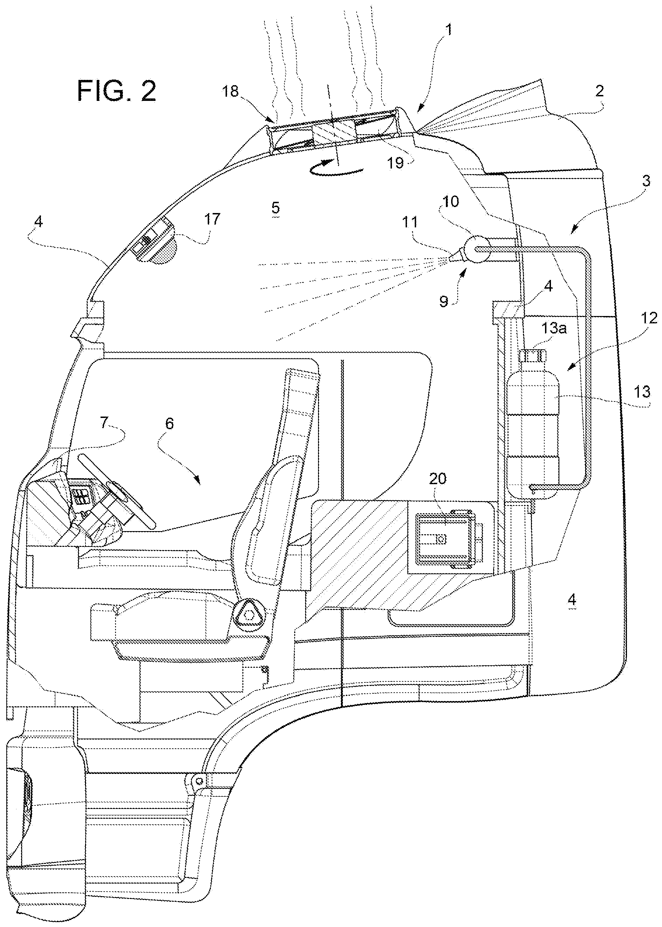

In Figure 2, the cab of a heavy-duty vehicle 2 such as a truck comprising a sanitization system 3 according to this invention is identified, as a whole, with the reference number 1.

As illustrated in Figure 2, the vehicle 2 comprises a number of walls 4 defining a housing portion 5 such as a cabin 1 configured to accommodate one or more passengers and housing, as known, a driver's seat 6 and a dashboard 7 located in front of the driver's seat 6 and defining support for a variety of driving control elements, such as a steering system, buttons, or screens.

The cab 1 is carried by a vehicle frame, not illustrated, which is movable across the ground, as known, and therefore not further described for brevity.

According to the invention, the vehicle 2 comprises a sanitization system 3 configured to sanitize the housing 5 using fluid.

Advantageously, the sanitization system 3 is integrated within the walls 4 defining the housing 5.

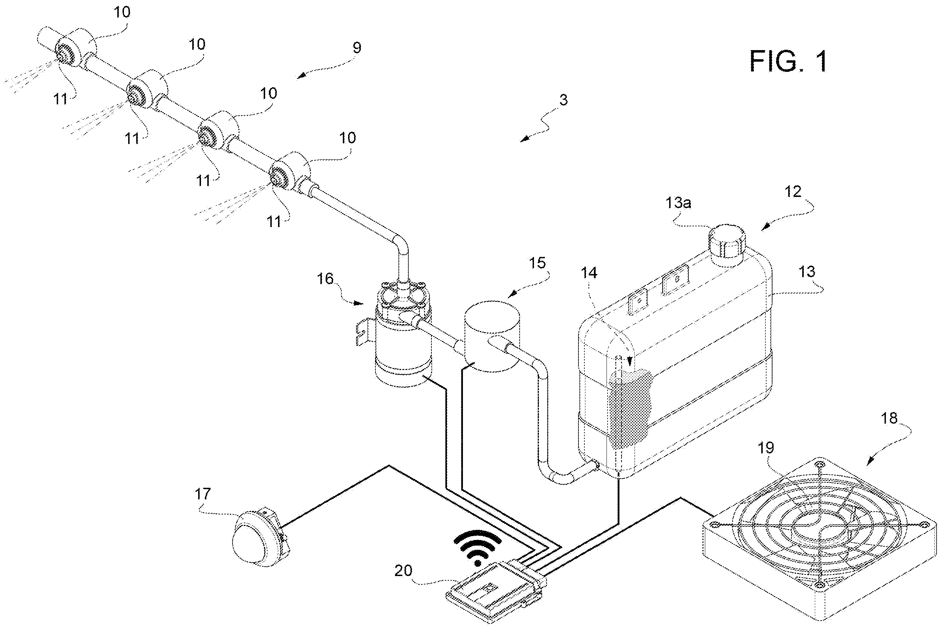

As illustrated in greater detail in Figure 1, the sanitization system 3 essentially comprises diffuser means 9 configured to spray a high-pressure sanitizing fluid contained in a sanitizing fluid source 12.

Advantageously, the diffuser means 9 comprise a number of nebulization points 10 each fluidically connected to the fluid source 12 and, in the described embodiment, fluidically in series with each other.

According to the illustrated embodiment, referring to Figure 2, this number of nebulization points 10 is carried by an internal wall 4 of the cab 1 in an elevated position so that the fluid that they nebulized can reach the whole housing 5 space.

Advantageously, each nebulization point 10 is guidable so that the position of the fluid that it nebulizes can vary spatially so as to reach each point of the cab 1.

Advantageously, each nebulization point 10 comprises a nozzle 11 configured to define a particular sanitizing fluid nebulization pattern. Each nozzle 11 can, preferably, be selectively mounted at the respective nebulization point 10.

These nozzles 11 may, preferably, be fixed-jet nozzles and may be replaced with nozzles of a size suitable for the size of the housing 5 or variable-jet nozzles configured to vary their flow rate according to an appropriate control signal.

The sanitizing fluid source 12 is preferably a tank 13, more preferably integrated within one of the walls 4, and provided with an opening 13a accessible from the outside to allow it to be filled, either directly, as in the illustrated example, or via a special duct, not illustrated.

The source 12 may advantageously be provided with level sensor means 14 configured to detect the level of sanitizing fluid contained within the tank 13.

The sanitizing fluid may be of any type suitable for sanitizing the housing 5, i.e. for eliminating pathogens such as viruses, bacteria, fungi and mould, such as, for example, hydrogen peroxide or any other known type of sanitizing fluid.

The sanitization system 3 further comprises pumping means 15 fluidically interposed between the source 12 and the diffuser means 9 configured to suck the sanitizing fluid from the tank 13 and to send it to the diffuser means 9. Advantageously, these pumping means 15 comprise an electric pump, powered at 12 V or 24 V depending on the power supply type of the vehicle 2. Optionally, the pumping means 15 may be provided with a special converter/inverter configured to vary the power supply voltage to the correct voltage for the type of pump installed.

The pumping means 15 are preferably configured to vary the pressure of the sanitizing fluid relative to the housing pressure within the tank 13.

The sanitization system 3 may comprise heating means 16 fluidically interposed between the pumping means 15 and the diffuser means 9 to heat the sanitizing fluid to a predetermined temperature, if required by the type of sanitizing fluid. Advantageously, these heating means 16 may comprise a heat exchanger, for example an electric one.

The sanitization system 3 may comprise sensor means 17 configured to detect the presence of people inside the housing 5. These sensor means 17 can, advantageously, comprise a volumetric sensor or a thermal video camera.

Optionally, the sensor means 17 may also be configured to allow the detection of the body temperature of the occupants by managing the tracking and acquisition of the daily data of the fleet manager with respect to risk indicators that may require sanitization at close time intervals.

The sanitization system 3 also comprises ventilation means 18 configured to selectively enable the fluidic communication of the housing 5 with the external environment. In the described embodiment, these ventilation means 18 comprise forced ventilation means 19 such as an electronically controlled fan, preferably when switched on or off, and when speed is controlled (air flow rate).

Advantageously, the sanitization system 3 comprises an electronic unit 20 electronically connected to the diffuser means 9, to the sensor means that the sanitization system 3 may be provided with, and to the ventilation means 18 and comprising processing means configured to receive an activation signal for the sanitization system 3 and to control, as a consequence, the operation of the diffuser means 9 and of the ventilation means 18, taking into account, when present, the sensor means described above.

In particular, the electronic unit 20 can be made separately or integrated into the vehicle 2 ECU.

The processing means are configured to store, or to receive as input, data relating to the size of the housing 5 and of the type of sanitizing fluid in order to calculate the quantity of the latter to be nebulized inside the housing 5.

Advantageously, the electronic unit 20 comprises telecommunication means configured to enable the remote control of the sanitization system 3, for example using a remote control, for example using vehicle fleet control software or, again, using a software application that can be installed on a smartphone.

Again, the electronic unit 20 may be electrically connected to a special button or screen function that can be selected by the user and activated once they have left the housing 5, a state that can be detected using the sensor means 17.

The operation of the embodiment of the sanitization system 3 described above is the following.

When the driver has finished their route and has exited the passenger compartment, they can activate the sanitization, as described above, using one of the remote/in-cab control systems for the sanitization system 3. This operation can also be carried out by a management plant for the vehicles 1, for example those parked in the fleet carpark.

Once the sanitization cycle has been activated, the electronic unit 20 activates a verification step to verify the operation of its elements, such as the pumping means 15, the ventilation device 18, or, if present, the heating means 16, or using the level sensor means 14, the verification of the presence of enough sanitizing fluid in the tank 13.

If an anomaly is detected, it is indicated using a light and/or sound indicator in the vehicle 2 and/or a remote management software/app notification.

If no anomalies are detected, the electronic unit 20, having stored data relating to the type of sanitizing fluid and the size of the housing 5, launches a fluid distribution step. This, therefore, controls the pumping means 15 so that they draw a predetermined quantity of sanitizing fluid from the source 12 and send it to the diffuser means 9 for dispersing it in the housing 5 using the nozzles 11.

If the latter are variable jet nozzles, the electronic unit 20 can adjust the opening so as to vary the nebulization thereof, according to a precise sanitizing fluid distribution cycle, depending on the size of the housing 5 and on the type of sanitizing fluid.

If it is necessary, based on the type of sanitizing fluid, to raise the temperature thereof before sending it to the diffuser means 9, the electronic unit 20 controls the heating means 16 so that they bring the sanitizing fluid to a predetermined temperature before nebulizing them using the diffuser means 9.

Once the fluid distribution cycle is complete, the electronic unit 20 checks that a predetermined time for allowing the sanitizing fluid to act has passed and checks the ventilation devices 18 so as to enable the gas residues of the nebulized fluid to escape into the environment and the housing 5 to be aired.

In the example described, the electronic unit 20 activates the forced ventilation means 19 so as to force the escape of the gas residues of the sanitizing fluid into the external environment and, thus, to air the housing 5.

In parallel to what is described above, the electronic unit 20 performs a continuous check that no living being enters inside the housing 5 using, for example, the sensor means 17 or by detecting the opening of the cab 1 doors. If this condition occurs, the sanitization is interrupted and the ventilation means 18 are immediately activated.

The electronic unit 20 can advantageously store the sanitization cycles successfully performed and communicate them to the remote management system by storing the date and time of the sanitization that took place.

In light of the above, it's clear that this invention also relates to a method of sanitizing a housing 5 for a vehicle 1 as described above and comprising the steps of:
- receiving a request to sanitize a vehicle 1 housing 5;
- activating a verification step for the sanitization system 3 functions;
- if the verification step reveals an anomaly, reporting this anomaly via IT and/or acoustic and/or visual notification;
- if the verification step does not detect any anomalies, proceeding with a step for distributing nebulized sanitizing fluid inside the housing 5;
- at the end of the fluid distribution step, after a predetermined waiting time, activating the ventilation means 18 to allow the housing 5 to be aired.

The method also comprises an additional step, in parallel to those listed above, of verifying whether there is a living being inside the housing 5, if this is verified the sanitization is stopped and the ventilation means 18 are operated.

The anomaly check step comprises at least one of the following steps:
- verification of the operation of the pumping means 15 and/or ventilation means 18 and/or heating means 16; and/or
- verification of the fluid level inside the tank 13.

The fluid distribution step has a predetermined duration depending on the type of sanitizing fluid and on the dimensions of the housing 5 according to what is processed by the electronic unit 20. In particular, the electronic unit 20 can also control the orientation of the nebulization points 10, i.e. the nozzles 11, depending on the size and arrangement of the housing 5.

Similarly, the wait before activating the ventilation means 18 has a predetermined duration depending on the type of sanitizing fluid to eliminate the pathogens concerned.

The method can also comprise the step of storing the date and time of the housing 5 sanitization that has occurred.

The advantages of a vehicle 2 provided with a sanitization system 3 according to this invention are clear from the above.

Thanks to the sanitization system 3, it is possible to automate and reduce the sanitization time of the vehicular housing 5, making it possible to sanitize effectively and safely.

The automatic system consumes little energy and can be totally controlled remotely, checking the operation thereof and the history of activations.

In addition, the proposed system is independent of the vehicle type and sanitizing liquid, and can be easily applied by varying the supply time for the sanitizing liquid inside the housing.

In addition, it is possible to easily integrate the electronic unit with the other control systems for the vehicle or vehicle fleet, by making the latter more versatile.

Thanks to the use of nozzles 11 that can be separated, it is possible to easily replace the flow rate of the diffuser means 9 by modifying the capacity of the sanitization system 3 depending on different operational needs.

Finally, it is clear that the vehicle 2 provided with a sanitization system 3 according to this invention can be modified, and variations can be made thereof, without, however, departing from the scope of protection as set forth in the claims.

For example, it is clear that although a cab 1 is illustrated, it is possible to use the sanitization system 3 in any vehicle housing 5 that can be used by vehicle operators.

Again, the number of devices, shapes, and arrangements described here may vary as a function of the type of vehicle with which they are associated.

As disclosed in the description, some of the functional elements of the sanitization system 3 may be optional, like the corresponding steps of the corresponding control method.

## Claims

1. Vehicle (1) comprising a plurality of walls (4) defining a housing (5), said vehicle (1) comprising a sanitization system (3) for said housing (5) comprising diffuser means (9), a source (12) of sanitizing fluid, ventilation means (18) and an electronic unit (20), said electronic unit (20) being configured to allow the passage of said sanitizing fluid from said source (12) to said diffuser means (9 ), said diffuser means (9) nebulizing said sanitizing fluid coming from said source (12) inside said housing (5), **characterized in that** said electronic unit (20) is configured to activate said ventilation means (18) to allow the airing of said housing (5) following the nebulization of said sanitizing fluid inside said housing (5).

2. Vehicle according to claim 1, wherein said source (12) comprises a tank (13) provided with fluid sensor means (14) configured to detect a level of sanitizing fluid in said tank (13).

3. Vehicle according to one of the preceding claims, comprising pumping means (15) fluidly interposed between said source (12) and said diffuser means (9) and configured to control the passage of said fluid from said source (12) to said diffuser means (9).

4. Vehicle according to claim 3, comprising heating means (16) fluidly interposed between said pumping means (15) and said diffuser means (9).

5. Vehicle according to one of the preceding claims, wherein said ventilation means (18) comprise forced ventilation means (19).

6. Vehicle according to one of the preceding claims, comprising sensor means (17) configured to detect the presence of a living being inside said housing (5), said sensor means (17) being electrically connected to said electronic unit (20) which is configured to prevent the nebulization of said sanitizing fluid and the activation of said ventilation means (18) if a living being is identified inside said housing (5).

7. Vehicle according to one of the preceding claims, wherein said diffuser means (9) comprise a plurality of nebulization points (10) each of said nebulization points (10) being fluidly connected to said source (12) of sanitizing fluid.

8. Vehicle according to claim 7, wherein said nebulization points (10) each comprise a nozzle (11) that can be selectively mounted in the respective nebulization point (10).

9. Vehicle according to claim 7 or 8, wherein each nebulization point (10) can be oriented.

10. Vehicle according to one of the preceding claims, wherein said electronic unit (20) comprises processing means configured to calculate the quantity of sanitizing fluid to be sprayed inside said housing 5 according to the type of sanitizing fluid and dimensions of said housing 5, said values being stored or insertable in said electronic unit (20).

11. Vehicle according to one of the preceding claims, wherein said electronic unit (20) comprises telecommunication means configured to allow remote control thereof.

12. Method of sanitizing a housing (5) of a vehicle (1) according to one of the preceding claims and comprising the steps of:
• receive a request to sanitize a vehicle (1) housing (5);
• activate a verification step for the functionality of the sanitization system (3);
• if the verification step reveals an anomaly, report this anomaly via IT and / or acoustic and / or visual notification;
• if the verification step does not detect any anomalies, proceed with a step for distributing nebulized sanitizing fluid inside the housing (5);
• at the end of the fluid distribution step, after a predetermined waiting time, activation of the ventilation means (18) to allow the housing (5) to be aired.

13. Sanitization method according to claim 12, further comprising a verification step, in parallel with those listed above, said verification step detecting if a living being is inside the housing (5), if this condition is detected said fluid distribution step is interrupted and the ventilation means (18) are simultaneously activated.

14. Sanitization method according to claim 12 or 13, wherein the anomaly verification step comprises at least one of the following steps:
• • verification of the operation of the pumping means (15) and/or ventilation means (18) and/or heating means (16); and/or
• • verification of the fluid level inside the tank (13).

15. Sanitization method according to one of claims 12 to 14, in which the fluid distribution step has a predetermined duration according to the type of sanitizing fluid and the size of the housing (5).

16. Sanitization method according to one of claims 12 to 15, further comprising a step for storing the date and time of a successful sanitization of the housing (5).

## Patentansprüche

1. Fahrzeug (1), umfassend eine Mehrzahl von Wänden (4), die ein Gehäuse (5) begrenzen, welches Fahrzeug (1) ein Reinigungssystem (3) für das Gehäuse (5) umfasst, umfassend Diffusormittel (9), eine Quelle (12) eines Reinigungsfluids, Belüftungsmittel (18) und eine elektronische Einheit (20), welche elektronische Einheit (20) dazu ausgebildet ist, den Durchlass des Reinigungsfluids von der Quelle (12) zu den Diffusormitteln (9) zu ermöglichen, welche Diffusormittel (9) das Reinigungsfluid, das von der Quelle (12) stammt, innerhalb des Gehäuses (5) zu versprühen, **dadurch gekennzeichnet, dass** die elektronische Einheit (20) dazu ausgebildet ist, die Belüftungsmittel (18) zu aktivieren, um die Belüftung des Gehäuses (5) nach dem Versprühen des Reinigungsfluids im Gehäuse (5) zu ermöglichen.

2. Fahrzeug gemäß Anspruch 1, bei welchem die Quelle (12) einen Tank (13) umfasst, ausgestattet mit Fluidsensormitteln (14), die zur Detektion eines Pegels des Reinigungsfluids in dem Tank (13) ausgebildet sind.

3. Fahrzeug gemäß einem der vorhergehenden Ansprüche, umfassend Pumpmittel (15), die fluidtechnisch zwischen der Quelle (12) und den Diffusormitteln (9) angeordnet sind und dazu ausgebildet sind, den Durchlass des Fluids von der Quelle (12) zu den Diffusormitteln (9) zu steuern.

4. Fahrzeug gemäß Anspruch 3, umfassend Heizmittel (16), die fluidtechnisch zwischen den Pumpmitteln (15) und den Diffusormitteln (9) angeordnet sind.

5. Fahrzeug gemäß einem der vorhergehenden Ansprüche, bei welchem die Belüftungsmittel (18) Zwangsbelüftungsmittel (19) umfassen.

6. Fahrzeug gemäß einem der vorhergehenden Ansprüche, umfassend Sensormittel (17), ausgebildet zur Detektion des Vorhandenseins eines Lebewesens innerhalb des Gehäuses (5), welches Sensormittel (17) elektrisch mit der elektronischen Einheit (20) verbunden sind, welche dazu ausgebildet ist, das Versprühen der Reinigungsflüssigkeit und die Aktivierung der Belüftungsmittel (18) zu verhindern, falls ein Lebewesen innerhalb des Gehäuses (5) identifiziert wird.

7. Fahrzeug gemäß einem der vorhergehenden Ansprüche, bei welchem die Diffusormittel (9) eine Mehrzahl von Sprühpunkten (10) umfassen, wobei jeder dieser Sprühpunkte (10) mit der Quelle (12) des Reinigungsfluids fluidverbunden ist.

8. Fahrzeug gemäß Anspruch 7, bei welchem die Sprühpunkte (10) jeweils eine Düse (11) umfassen, die selektiv in dem jeweiligen Sprühpunkt (10) montiert ist.

9. Fahrzeug gemäß Anspruch 7 oder 8, bei welchem jeder Sprühpunkt (10) ausgerichtet werden kann.

10. Fahrzeug gemäß einem der vorhergehenden Ansprüche, bei welchem die elektronische Einheit (20) Verarbeitungsmittel umfasst, ausgebildet zur Berechnung der Menge des innerhalb des Gehäuses (5) zu versprühenden Reinigungsfluids entsprechend der Art des Reinigungsfluids und der Abmessungen des Gehäuses (5), welche Werte in der elektronischen Einheit (20) gespeichert sind oder in diese eingebbar sind.

11. Fahrzeug gemäß einem der vorhergehenden Ansprüche, bei welchem die elektronische Einheit (20) Telekommunikationsmittel umfasst, ausgebildet zur Ermöglichung einer Fernsteuerung derselben.

12. Verfahren zur Reinigung eines Gehäuses (5) eines Fahrzeugs (1) gemäß einem der vorhergehenden Ansprüche, umfassend die folgenden Schritte:
- Empfang einer Aufforderung zur Reinigung eines Gehäuses (5) eines Fahrzeugs (1);
- Aktivierung eines Verifikationsschritts für die Betriebsfähigkeit des Reinigungssystem (3);
- falls der Verifikationsschritt eine Anomalie offenbart: Berichten dieser Anomalie über eine IT- und/oder eine akustische und/oder eine visuelle Mitteilung;
- falls der Verifikationsschritt keine Anomalien offenbart: Fortsetzung mit einem Schritt zur Verteilung versprühter Reinigungsflüssigkeit innerhalb des Gehäuses (5);
- am Ende des Fluidverteilungsschritts, nach einer vorbestimmten Wartezeit, Aktivierung der Belüftungsmittel (18), so dass das Gehäuse (5) belüftet werden kann.

13. Reinigungsverfahren gemäß Anspruch 12, ferner umfassend einen Verifikationsschritt, parallel zu den oben angegebenen Schritten, welcher Verifikationsschritt feststellt, ob sich ein Lebewesen innerhalb des Gehäuses (5) befindet, und falls dieser Zustand detektiert wird, wird der Fluidverteilungsschritt unterbrochen und die Belüftungsmittel (18) werden gleichzeitig aktiviert.

14. Reinigungsverfahren gemäß Anspruch 12 oder 13, bei welchem der Anomalie-Verifikationsschritt zumindest einen der folgenden Schritte umfasst:
- Bestätigung des Betriebs der Pumpmittel (15) und/oder Belüftungsmittel (18) und/oder der Heizmittel (16); und/oder
- Bestätigung des Fluidpegels innerhalb des Tanks (13).

15. Reinigungsverfahren gemäß einem der Ansprüche 12 bis 14, in welchem der Fluidverteilungsschritt eine vorbestimmte Dauer entsprechend der Art des Reinigungsfluids und der Größe des Gehäuses (5) hat.

16. Reinigungsverfahren gemäß einem der Ansprüche 12 bis 15, ferner umfassend einen Schritt der Speicherung des Datums und der Zeit einer erfolgreichen Reinigung des Gehäuses (5).

## Revendications

1. Véhicule (1) comprenant une pluralité de parois (4) définissant un logement (5), ledit véhicule (1) comprenant un système de désinfection (3) pour ledit logement (5) comprenant des moyens de diffusion (9), une source (12) de fluide désinfectant, des moyens de ventilation (18) et une unité électronique (20), ladite unité électronique (20) étant configurée pour permettre le passage dudit fluide désinfectant de ladite source (12) auxdits moyens de diffusion (9), lesdits moyens de diffusion (9) nébulisant ledit fluide désinfectant provenant de ladite source (12) à l'intérieur dudit logement (5), **caractérisé en ce que** ladite unité électronique (20) est configurée pour activer lesdits moyens de ventilation (18) pour permettre l'aération dudit logement (5) suite à la nébulisation dudit fluide désinfectant à l'intérieur dudit logement (5).

2. Véhicule selon la revendication 1, dans lequel ladite source (12) comprend un réservoir (13) doté de moyens de détection de fluide (14) configurés pour détecter un niveau de fluide désinfectant dans ledit réservoir (13).

3. Véhicule selon l'une des revendications précédentes, comprenant des moyens de pompage (15) interposés fluidiquement entre ladite source (12) et lesdits moyens de diffusion (9) et configurés pour commander le passage dudit fluide de ladite source (12) auxdits moyens de diffusion (9).

4. Véhicule selon la revendication 3, comprenant des moyens de chauffage (16) interposés fluidiquement entre lesdits moyens de pompage (15) et lesdits moyens de diffusion (9).

5. Véhicule selon l'une des revendications précédentes, dans lequel lesdits moyens de ventilation (18) comprennent des moyens de ventilation forcée (19).

6. Véhicule selon l'une des revendications précédentes, comprenant des moyens de détection (17) configurés pour détecter la présence d'un être vivant à l'intérieur dudit logement (5), lesdits moyens de détection (17) étant électriquement connectés à ladite unité électronique (20) qui est configurée pour empêcher la nébulisation dudit fluide désinfectant et l'activation desdits moyens de ventilation (18) si un être vivant est identifié à l'intérieur dudit logement (5).

7. Véhicule selon l'une des revendications précédentes, dans lequel lesdits moyens de diffusion (9) comprennent une pluralité de points de nébulisation (10), chacun desdits points de nébulisation (10) étant fluidiquement relié à ladite source (12) de fluide désinfectant.

8. Véhicule selon la revendication 7, dans lequel lesdits points de nébulisation (10) comprennent chacun une buse (11) qui peut être sélectivement montée dans le point de nébulisation (10) respectif.

9. Véhicule selon la revendication 7 ou 8, dans lequel chaque point de nébulisation (10) peut être orienté.

10. Véhicule selon l'une des revendications précédentes, dans lequel ladite unité électronique (20) comprend des moyens de traitement configurés pour calculer la quantité de fluide désinfectant devant être pulvérisée à l'intérieur dudit logement (5) selon le type de fluide désinfectant et les dimensions dudit logement (5), lesdites valeurs étant stockées ou pouvant être insérées dans ladite unité électronique (20).

11. Véhicule selon l'une des revendications précédentes, dans lequel ladite unité électronique (20) comprend des moyens de télécommunication configurés pour permettre une commande à distance de ceux-ci.

12. Méthode de désinfection d'un logement (5) d'un véhicule (1) selon l'une des revendications précédentes et comprenant les étapes de :
- réception d'une demande de désinfection d'un logement (5) de véhicule (1) ;
- activation d'une étape de vérification pour la fonctionnalité du système de désinfection (3) ;
- si l'étape de vérification révèle une anomalie, rapport de cette anomalie par le biais d'une notification par TI et/ou acoustique et/ou visuelle ;
- si l'étape de vérification ne détecte aucune anomalie, passage à une étape de distribution de fluide désinfectant nébulisé à l'intérieur du logement (5) ;
- à la fin de l'étape de distribution de fluide, après un temps d'attente prédéterminé, activation des moyens de ventilation (18) pour permettre l'aération du logement (5).

13. Méthode de désinfection selon la revendication 12, comprenant en outre une étape de vérification, en parallèle avec celles listées ci-dessus, ladite étape de vérification détectant si un être vidant est à l'intérieur du logement (5), si cette condition est détectée ladite étape de distribution de fluide est interrompue et les moyens de ventilation (18) sont simultanément activés.

14. Méthode de désinfection selon la revendication 12 ou 13, dans laquelle l'étape de vérification d'anomalie comprend au moins l'une des étapes suivantes :
- la vérification de l'opération des moyens de pompage (15) et/ou des moyens de ventilation (18) et/ou des moyens de chauffage (16) ; et/ou
- la vérification du niveau de fluide à l'intérieur du réservoir (13).

15. Méthode de désinfection selon l'une des revendications 12 à 14, dans laquelle l'étape de distribution de fluide présente une durée prédéterminée selon le type de fluide désinfectant et la taille du logement (5).

16. Méthode de désinfection selon l'une des revendications 12 à 15, comprenant en outre une étape de stockage de la date et de l'heure d'une désinfection réussie du logement (5).
